# EUROPEAN PATENT SPECIFICATION

(11) **EP 0 685 445 B1**
(45) Date of publication and mention of the grant of the patent: **22.10.1997**
(21) Application number: 95106652.1
(22) Date of filing: 03.05.1995
(51) Int. Cl.: C07C 53/08, C07C 51/47

(54) **Process for removing iodine-containing compounds from acetic acid**
Verfahren zur Entfernung von Jod enthaltenden Verbindungen aus Essigsäure
Procédé d'élimination du composés renfermant l'iode à partir d'acide acétique

(30) Priority: 31.05.1994 US 251226
(43) Date of publication of application: 06.12.1995
(73) Proprietor: EASTMAN CHEMICAL COMPANY, Kingsport, TN 37660 (US)
(72) Inventor: Fillers, Carl Franklin, c/o Eastman Chemical Co., Kingsport, Tennessee 37662-5075 (US); Cook, Steven Leroy, c/o Eastman Chemical Co., Kingsport, Tennessee 37662-5075 (US)
(74) Representative: Wibbelmann, Jobst, Dr., Dipl.-Chem.

(56) References cited:
- EP-A- 0 544 496
- GB-A- 2 064 526
- US-A- 4 221 871

## Description

This invention pertains to a process for removing iodine-containing compounds from acetic acid. More specifically, this invention pertains to a process for reducing iodine-containing compounds present in acetic acid by contacting iodine-contaminated acetic acid with certain ion exchange resins.

Various processes for the preparation of carboxylic acids and anhydrides, including the coproduction of carboxylic acids and anhydrides or carboxylic anhydrides and alkylidene dicarboxylates, by the catalytic carbonylation of alcohols, ethers, esters and/or olefins have been described extensively in the literature and, in some cases, have been used on a commercial scale. Typically, these carbonylation processes are carried out in the presence of a Group VIII metal and an iodine-containing compound such as, but not limited to, hydrogen iodide, an alkyl iodide such as methyl iodide, a phosphonium iodide, an alkali metal iodide, or an iodide salt of a number of other catalyst or promoter components. See, for example, S.W. Polichnowski, J. Chem. Educ., 1986, 63, 206 and U.S. Patents 3,769,329, 4,374,070, 4,661,631 and 4,994,608. The carboxyl product-containing reactor effluents from such carbonylation processes commonly are subjected to conventional separation procedures, such as fractional distillation, which allows the recovery and recycle of both the volatile and non-volatile iodine-containing compounds. Indeed, the economic operation of such processes depends, in part, upon such an effective recycle. In spite of the efficiency of most separation methods, however, small amounts of iodine-containing compounds, e.g., up to about 250 parts per million by weight (ppm) [I], typically are contained in the product. For certain end uses, e.g., acetic acid utilized in the manufacture of vinyl acetate, purchasers require that the iodine content of carboxyl compounds be extremely low, e.g., <20 parts per billion by weight (ppb).

Canadian Patent 1,234,149 (equivalent to EP 143,179) describes the treatment of acetic acid or acetic anhydride at 50 to 200°C, preferably 80 to 140°C, with hydrogen in the presence of a Group VIII noble metal such as ruthenium, osmium, iridium or, preferably rhodium, palladium or platinum finely distributed on a silica or alumina support. This treatment is said to reduce the iodine content, present as alkyl or aryl iodides and hydrogen iodide or phosphonium iodide salts, to acceptable, e.g., <20 ppb, levels. The residence time is 0.2 to 6 hours and treatment is carried out at a pressure of 0.5 to 10 bars. The catalyst is preferably separated by filtration and is discarded when its capacity is exhausted. A comparative example reveals that iodine removal is not acceptable when hydrogen is omitted.

Canadian Patent 1,279,655 (equivalent to EP 217,182) discloses an improvement to the process disclosed in CA 1,234,149 which involves the addition of carbon monoxide to the gaseous feed to overcome a serious defect. Apparently, in the absence of added carbon monoxide, catalyst activity decreases relatively rapidly, and efficient iodine removal then requires that the operating temperature of the catalyst bed be increased. At these increased temperatures excessive by-product formation (acetaldehyde, acetic acid and ethylidene diacetate when the stream being treated contains acetic anhydride) occurs. It was found that if the gas fed to the treating bed contains carbon monoxide then the formation of these reduced by-products could be minimized.

The use of a heterogeneous noble metal catalyst according to the known processes cited above presents at least two significant disadvantages. The first is the cost of the noble metal catalysts. This is of particular concern as the catalyst is discarded when exhausted. Furthermore, in the above-described process, in order to maintain catalyst activity and avoid side-reactions, both hydrogen and carbon monoxide must be present in the stream to be treated. This increases both the complexity of the system and its cost.

U.S. Patent 4,664,753 discloses a method for iodine removal from acetic acid, acetic anhydride and/or ethylidene diacetate streams at a temperature of 20 to 250°C. The disclosed method requires a two component system consisting of (i) a phosphine or amine and (ii) zinc or compounds of zinc copper, silver or cadmium. Treatment with this two-component system is followed by distillation. These chemical traps might be placed in a still bottom and continuously circulated through a reboiler. Contact (residence) times of 15 to 120 minutes are required and continuous replenishment is possible.

The process proposed in U.S. Patent 4,664,753 utilizes a chemical trap comprising a two-component system which degrades with time. The materials formed by the reaction of the chemical traps with iodine or organic iodine-containing species may not be stable to the conditions of prolonged use. Furthermore, prolonged residence of active components of the chemical traps in the reboiler of the distillation train likely would result in fouling.

U.S. Patent 4,615,806 discloses a method for removing iodide compounds from a non-aqueous medium, such as acetic acid. The medium is contacted with a macroreticulated strong-acid, e.g., sulfonic acid, cation exchange resin which is stable in the organic medium and has at least 1%, preferably 25 to 75%, of its active sites converted to the silver or mercury form. Regeneration of the resin and/or recovery of the metals are potential problems in the operation of the process of U.S. Patent 4,615,806.

Published European Patent Application Publication No. 544,496 describes the removal of halide contaminants from acetic acid. The halide removal process comprises contacting the halide-containing acetic acid with a polymeric resin having functional groups forming coordination complexes with one or more metal salts capable of reacting with halides to form metal halide precipitates. The metal complexed polymeric resin preferably is poly-4-vinylpyridine, polyphenylene sulfide or polybenzimidazole complexed with a silver, mercury, copper or lead salt.

Basic ion exchange copolymers derived from vinyl-pyridine and divinylbenzene have been used to remove phenolic impurities in hydrogen peroxide (U.S. Patent 4,975,266) and to recover phenols (German Patent 2,947,765), recover carboxylic acids (German Patent 3,043,766 and in American Laboratory, 92, 94-9 (1990)), remove molecular iodine (Japanese Patent 51148693, 50113560), and remove sulfur dioxide from air (German Patent 2,164,261). It is also useful for chromium adsorption (Japanese Patent 51114389). The prior art does not disclose or teach that such vinylpyridine/divinylbenzene copolymers would exhibit selectivity for adsorbing organo-iodine and iodide salts as well as molecular iodine present in a carboxylic acid such as acetic acid.

The process of the present invention provides an efficient means for the removal of iodine and its compounds from acetic acid streams. The present invention provides a process for the reduction in the iodine content of a crude acetic acid stream comprising (i) acetic acid and (ii) a plurality of iodine-containing compounds selected from the group consisting of organo-iodides, iodide salts, hydrogen iodide and molecular iodine, which comprises the steps of:
(1) feeding the crude acetic acid stream to an iodine removal zone wherein the stream is contacted with an ion exchange resin wherein the ion exchange functionality comprises unmodified residues of vinylpyridine; and
(2) removing from the iodine removal zone a refined acetic acid stream containing less iodine than the crude acetic acid stream.

The crude, iodine-containing, acetic acid stream normally is obtained from a carbonylation production system wherein methanol, dimethyl ether, methyl acetate and/or methyl iodide is contacted with carbon monoxide in the presence of a Group VIII metal, elemental iodine and/or an iodine compound and, optionally, one or more promoters or primary catalyst stabilizers. The use of iodine in the carbonylation process leads to the formation of various iodine species, depending in part upon the various materials which may be included in the particular catalyst system or combination in use. Such iodine-containing impurities may comprise iodine; one or more organo-iodides such as an alkyl iodide, e.g., methyl or ethyl iodide, an aryl iodide, e.g., iodobenzene, an alkyl iodoacetic acid, e.g., methyl iodoacetate, or an iodoalkyl acetate, e.g., iodomethyl acetate; hydrogen iodide; an iodide of a quaternary ammonium or phosphonium compound; an iodide salt of a transition metal such as rhodium, iron, chromium, nickel, molybdenum, etc.; an iodide salt of an alkali or alkaline earth metal such as sodium, lithium, potassium, beryllium, magnesium, or calcium. Unless stated otherwise, all references herein to "iodine" include all forms of iodine such as elemental iodine and the iodide compounds mentioned above.

The iodine content of the crude acetic acid stream may be as high as 250 ppm but normally will be in the range of 500 ppb to 10 ppm. Generally, the degree of iodine removal depends on a plurality of factors such as the concentration of iodine in the crude acetic acid stream, the particular iodine-containing compounds present, the time the acetic acid stream is in contact with the vinylpyridine/divinylbenzene ion exchange resin, the temperature of the acetic acid stream and/or the ion exchange resin. Thus, the degree of iodine removal which may be achieved by the use of the process of the present invention may be characterized by (1) the concentration of iodine in the crude acetic acid stream fed to the process relative to (2) the concentration of iodine in the refined acetic acid produced by the process. The value of (1)/(2) may be in the range of 12 to 1 but more typically will be in the range of 8 to 1. The amount of iodine, including iodine-containing compounds, which can be removed from acetic acid by the process of the present invention is not sufficient to provide a purified acetic acid for general use. Therefore, the present iodine-removal process normally will be used in conjunction with another purification process, such as one of those described in the prior art cited hereinabove.

Other materials (in addition to those mentioned above) which may be present in very low concentrations in the crude acetic acid stream used as the feedstock in the process of this invention include methanol, methyl acetate, dimethyl ether, methyl iodide, water and/or other materials endogenous to the catalytic carbonylation processes or decomposition products of such materials. Normally, acetic acid will constitute at least 95 weight percent, preferably at least 98 weight percent, of the crude acetic acid stream.

The ion exchange resins which may be used in the process of the invention comprise macroreticular polyvinylpyridine polymers cross-linked with difunctional monomers such as divinylbenzene. Examples of such resins are disclosed in U.S. Patents 4,221,871, 4,224,415, 4,256,840, 4,297,220, and 4,382,124. The ion exchange resin preferably comprise a polymer of unmodified 4-vinylpyridine residues and divinylbenzene residues. The vinylpyridine residues are characterized herein as unmodified which means that the tertiary amine functionality provided by the pyridine residues is not bound to a metal as described in U.S. Patent 4,615,806 and Published European Patent Application Publication No. 544,496 referred to hereinabove. A specific ion exchange resin which has been found to be useful is Reillex 425 resin available from Reilly Industries, Inc. The ion exchange resin may be used in the form of a powder which may be admixed intimately with a crude acetic acid stream and then separated by conventional liquid-solid separation techniques such as filtration or centrifugation. However, the ion exchange resin preferably is employed in the form of beads or granules of sufficient size to permit the acetic acid to pass over and through one or more beds of the particulate resin. A sphere or particle size of 18 to 50 mesh is typical.

The purification process of the invention may be carried out in the liquid or vapor phase over a temperature range of 80 to 160°C. Since vapor phase operation does not provide the high contact time and substrate density provided by liquid phase operation, vapor phase operation is not preferred although the use of higher temperatures may permit the removal of one or more iodine-containing compounds which are not affected by the resin at lower temperatures.
Therefore, the process preferably is carried out in the liquid phase at a temperature of 80 to 110°C with temperatures in the range of 95 to 105°C being especially preferred. Pressure is not an important element of the present invention and, therefore, pressures moderately above or below atmospheric or ambient pressure may be used.

The volume of the ion exchange resin required to purify a given amount of crude acetic acid or can vary significantly depending on a number of variables such as the iodine content of the crude acetic acid stream to be purified, the iodine concentration desired in the purified acetic acid, the particular ion exchange resin employed and the mode of operation. To achieve a significant reduction in the iodine concentration of the crude acetic acid stream fed to ion exchange resin, the liquid hourly space velocity (LHSV), i.e., the unit volume of crude acetic acid feed per unit volume of ion exchange resin per hour, usually should not exceed 1000. For purifying crude acetic acid having an iodine content of 3 to 12 ppm iodine, the LHSV normally should be in the range of 100 to 500, preferably in the range of 200 to 300.

The pyridine-containing ion exchange resin may be regenerated periodically by contacting it with a solution of a base such as an aqueous solution of an alkali metal hydroxide or carbonate, especially sodium hydroxide. The amount of the alkali-containing regeneration solution employed may be in the range of 1 to 20 parts by weight of regeneration solution per part by weight ion exchange resin. The contact time between the ion exchange resin and the regeneration solution normally is at least 60 seconds, usually from 5 to 10 minutes.

The iodine removal process of the present invention is further illustrated by the following examples. The iodine-containing acetic acid used in the examples was produced by the carbonylation of a mixture of methanol and methyl iodide in the presence of a catalyst system comprising a Group VIII metal and triphenyl phosphine. As a result of decomposition of a portion of the triphenyl phosphine, most portions of the acetic acid purified contained some iodobenzene.

The purified acetic acid produced in the examples is analyzed for iodine content by ion chromatography (sensitive to 10 ppb iodine). Since iodine present in the form of iodobenzene is not detected by ion chromatography analysis, samples of the purified acetic acid are pretreated to convert the iodobenzene to iodine compound(s) which are detected by ion chromatography. The crude acetic acid employed in the examples typically consists of 99+ weight percent acetic acid. Total iodine content of the crude acetic is determined by X-ray and/or ion chromatography analyses.

Examples 1-13 illustrate liquid phase operation of the iodine removal process wherein crude acetic acid was fed at a rate of 200 mL per hour to a glass column having an inside diameter of 1 inch (2.54 cm) and a length of 4 feet (1.2 meter). The glass column contained a 3-foot (0.9 meter) long bed consisting of 290 g (dry basis) of Reillex 425 ion exchange resin. This resin was used without regeneration in all of the procedures of Examples 1-13. The resin was maintained at a temperature of 103°C by feeding steam to a jacket surrounding the column.

The total weight (g) and total iodine content (ppm) of the crude acetic acid fed to the ion exchange resin bed in each of Examples 1-13 and the total iodine content (ppm) of the acetic acid effluent (purified acetic acid) from the resin bed are shown in Table I.

**TABLE I**

| Example | Acetic Acid Feed | | Purified Acetic Acid |
|---|---|---|---|
| | Total Iodine | Total Weight | Total Iodine |
| 1 | 3 | 3484 | 1.03 |
| 2 | 3 | 8596 | 2.00 |
| 3 | 8 | 7126 | 0.34 |
| 4 | 4 | 7962 | 1.58 |
| 5 | 8 | 7947 | 0.89 |
| 6 | 8 | 14865 | 1.17 |
| 7 | 11 | 8020 | 1.55 |
| 8 | 10 | 6911 | 1.15 |
| 9 | 12 | 7995 | 0.94 |
| 10 | 8 | 6917 | 1.44 |
| 11 | 9 | 8047 | 1.15 |
| 12 | 4 | 15717 | 2.01 |
| 13 | 8 | 8049 | 0.17 |

The apparent lack of correlation between the iodine content of the acetic acid feed and the iodine content of the purified acetic acid is due to the presence of varying amounts of iodobenzene in the feed material. Vinylpyridine/divinylbenzene ion exchange resins are not effective for the removal of aromatic iodides such as iodobenzene. Gas and liquid chromatographic analyses of samples of the acetic acid purified in some of Examples 1-13 failed to detect any iodomethyl acetate or methyl iodoacetate and showed that iodobenzene and methyl iodide made up most of the iodine content of the purified acetic.

Examples 14 illustrates gas phase operation of the iodine removal process wherein acetic acid containing one or more iodine-containing compounds was pumped to an evaporator comprising a plurality of loops of tubing constructed of 316 stainless steel and heated electrically. The hot vapor from the evaporator then was mixed hydrogen and nitrogen diluent gas. The gas flow rates were measured by Fisher-Porter rotameters calibrated at standard temperature and pressure by water displacement. The mixture of hydrogen and nitrogen was prepared by continuously mixing the pure gases to the desired percentage of hydrogen by passage through a mixing tee. The gas mixture comprising approximately 55 volume percent acetic acid, 5 volume percent hydrogen and 40 volume percent nitrogen was fed at a rate of 150 mL per minute to an iodine removal zone which consisted of a 316 stainless steel tube 10 inches (25.4 cm) x 0.5 inches (1.27 cm) diameter containing 12.3 g (dry basis) of Reillex 425 ion exchange resin. The approximate active volume of the resin bed was 32 mL. The rate at which acetic acid was fed to the iodine removal zone varied from 24 to 30 mL per hour due to mechanical variances of the feed pump. The iodine removal zone was electrically heated to 160°C which was controlled to ±2°C by a thermocouple inserted near the middle of the bed and connected to a temperature controller. All areas of the iodine removal system were electrically heated to prevent acetic acid from condensing in various cool spots. The effluent from the iodine removal zone was collected in a graduated cylinder and the off-gas passed through a water-cooled condenser to capture most of the acid.

Example 14 involved feeding over an extended period of operation a vapor of iodine-containing acetic acid by means of the apparatus described above to an iodine removal zone containing Reillex 425 ion exchange resin. During the first 64 hours of operation, the acetic acid feed contained 8 ppm iodine prepared from hydrogen iodide and acetic acid. During the next 154 hours the acetic acid feed contained 30 ppm iodine and was produced by the carbonylation of a mixture of methanol and methyl iodide in the presence of a catalyst system comprising a Group VIII metal and triphenyl phosphine. During the last 78 hours of operation, the acetic acid feed contained 27 ppm iodine prepared from hydrogen iodide and acetic acid. After 296 hours of operation, the resin bed became plugged due to bonding of the resin beads or particles.

Samples of the effluent from the iodine removal zone were collected periodically and analyzed for iodine content according to the procedures referred to hereinabove. The times at which samples were taken, the iodine content (ppm) of the acetic acid fed to the iodine removal zone and the iodine content (parts per billion, ppb) of acetic acid effluent from the iodine removal zone and the rates (mL per hour) at which liquid acetic acid was fed to the iodine removal zone are set forth in Table II. Analyses of the samples collected during hours 64-218 did not determine the amount of iodobenzene present.

**TABLE II**

| Hours of Operation | Acetic Acid Feed Rate | Iodine Content of Acetic Acid | |
|---|---|---|---|
| | | Fed | Purified |
| 4 | 30 | 8 | <10 |
| 32 | 30 | 8 | <10 |
| 39 | 25 | 8 | <10 |
| 64 | 30 | 8 | <10 |
| 71 | 26 | 30 | 34 |
| 92 | 30 | 30 | 47 |
| 116 | 25 | 30 | 64 |
| 140 | 24 | 30 | 71 |
| 164 | 25 | 30 | 76 |
| 194 | 27 | 30 | 86 |
| 218 | 27 | 30 | 60 |
| 252 | 26 | 27 | 111 |
| 296 | 27 | 27 | 105 |

A 10 g sample of dry Reillex 425 ion exchange resin which had been allowed to swell overnight in acetic acid was placed in a 100-mL, glass-jacketed column. The column was maintained at 100-103°C with steam. Acetic acid containing from 60 to 3000 ppm iodine was fed to the resin bed at feed rate of 200 mL per hour per liter of resin until the iodine concentration in the bed effluent was the same as the iodine concentration in the crude acetic acid feed. The bed of ion exchange resin was washed successively with water to remove acetic acid, 5% aqueous sulfuric acid solution, water, 5% aqueous sodium hydroxide solution and water. These washings were analyzed for iodine and the total iodine capacity of the resin was determined. The resin was washed with acetic acid to remove the water and the above-described procedure was repeated. The 10-g resin bed was saturated with iodine and regenerated five times with no observed loss in iodine removal capacity. The iodine capacity of the resin was determine to be 0.857 to 0.907 g of iodine per g of dry resin. The sulfuric acid wash removed a small amount of the iodine and most of the metals from the bed, and the sodium hydroxide wash removed most of the remaining iodine.

## Claims

1. Process for the reduction in the iodine content of a crude acetic acid stream comprising (i) acetic acid and (ii) a plurality of iodine-containing compounds selected from the group consisting of organo-iodides, iodide salts, hydrogen iodide and molecular iodine, which comprises the steps of:
(1) feeding the crude acetic acid stream to an iodine removal zone wherein the stream is contacted with an ion exchange resin wherein the ion exchange functionality comprises unmodified residues of vinylpyridine; and
(2) removing from the iodine removal zone a refined acetic acid stream containing less iodine than the crude acetic acid stream.

2. Process according to Claim 1 wherein the ion exchange resin is a polymer of unmodified 4-vinylpyridine residues and divinylbenzene residues.

3. Process according to Claim 1 which comprises the steps of:
(1) feeding the crude acetic acid stream to an iodine removal zone wherein the stream is contacted at a temperature of 80 to 110°C with an ion exchange resin wherein the ion exchange functionality comprises unmodified residues of vinylpyridine; and
(2) removing from the iodine removal zone a refined acetic acid stream containing less iodine than the crude acetic acid stream.
wherein the iodine content of the crude acetic acid is in the range of 500 parts per billion to 10 parts per million and the unit volume of crude acetic acid feed per unit volume of ion exchange resin per hour is in the range of 100 to 500.

## Patentansprüche

1. Verfahren zur Verringerung des Iod-Gehalts eines rohen Essigsäure-Stromes, der (i) Essigsäure und (ii) eine Mehrzahl von Iod-haltigen Verbindungen umfaßt, die aus der Gruppe ausgewählt sind, die aus Organoiodiden, Iodidsalzen, Iodwasserstoff und molekularem Iod besteht, welches die Schritte umfaßt:
(1) Zuführen des rohen Essigsäure-Stromes zu einer Iod-Entfernungszone, in der der Strom mit einem Ionenaustauscherharz in Kontakt gebracht wird, in dem die Ionenaustauscher-Funktionalität unmodifizierte Reste von Vinylpyridin umfaßt; und
(2) Entfernen eines verfeinerten Essigsäure-Stromes, der weniger Iod als der rohe Essigsäure-Strom enthält, aus der Iod-Entfernungszone.

2. Verfahren nach Anspruch 1, in dem das Ionenaustauscherharz ein Polymer von unmodifizierten 4-Vinylpyridin-Resten und Divinylbenzol-Resten ist.

3. Verfahren nach Anspruch 1, das die Schritte umfaßt:
(1) Zuführen des rohen Essigsäure-Stromes zu einer Iod-Entfernungszone, in der der Strom bei einer Temperatur von 80 bis 110°C mit einem Ionenaustauscherharz in Kontakt gebracht wird, in welchem die Ionenaustauscher-Funktionalität unmodifizierte Reste von Vinylpyridin umfaßt; und
(2) Entfernen eines verfeinerten Essigsäure-Stromes, der weniger Iod als der rohe Essigsäure-Strom enthält, aus der Iod-Entfernungszone,
in dem der Iod-Gehalt der rohen Essigsäure im Bereich von 500 Teilen pro Milliarde bis 10 Teilen pro Million liegt und das Einheitsvolumen an roher Essigsäure-Zufuhr pro Einheitsvolumen Ionenaustauscherharz pro Stunde im Bereich von 100 bis 500 liegt.

## Revendications

1. Procédé de diminution de la teneur en iode d'un courant d'acide acétique brut comprenant (i) de l'acide acétique et (ii) une pluralité de composés iodés sélectionnés dans le groupe constitué par des organo-iodures, des sels iodures, de l'iodure d'hydrogène et de l'iode moléculaire, lequel comprend les étapes consistant à :
(1) introduire le courant d'acide acétique brut dans une zone de séparation d'iode où le courant est mis en contact avec une résine échangeuse d'ions dont la fonctionnalité échangeuse d'ions comprend des résidus non modifiés de vinylpyridine ; et
(2) retirer de la zone de séparation d'iode un courant d'acide acétique raffiné contenant moins d'iode que le courant d'acide acétique brut.

2. Procédé selon la revendication 1, où la résine échangeuse d'ions est un polymère de résidus 4-vinylpyridine non modifiés et de résidus divinylbenzène.

3. Procédé selon la revendication 1, lequel comprend les étapes consistant à :
(1) introduire le courant d'acide acétique brut dans une zone de séparation d'iode où le courant est mis en contact à une température de 80 à 110°C avec une résine échangeuse d'ions dont la fonctionnalité échangeuse d'ions comprend des résidus non modifiés de vinylpyridine ; et
(2) retirer de la zone de séparation d'iode un courant d'acide acétique raffiné contenant moins d'iode que le courant d'acide acétique brut,
la teneur en iode de l'acide acétique brut étant comprise dans l'intervalle de 500 parties par milliard à 10 parties par million et le volume spécifique de la charge d'acide acétique brut par volume spécifique de résine échangeuse d'ions par heure étant de l'ordre de 100 à 500.
